# EUROPEAN PATENT SPECIFICATION

(11) **EP 1 700 579 B1**
(45) Date of publication and mention of the grant of the patent: **03.12.2008**
(21) Application number: 06004021.9
(22) Date of filing: 28.02.2006
(51) Int. Cl.: A61F 2/00

(54) **Surgical implant**
Chirurgisches Implantat
Implant chirurgical

(30) Priority: 11.03.2005 DE 102005012555
(43) Date of publication of application: 13.09.2006
(73) Proprietor: Johnson & Johnson Medical GmbH, 22851 Norderstedt (DE)
(72) Inventor: Walther, Christoph, 24568 Kattendorf (DE); Schuldt-Hempe, Barbara, 24576 Bad Bramstedt (DE); Fritz, Ingo, 22309 Hamburg (DE)
(74) Representative: UEXKÜLL & STOLBERG

(56) References cited:
- WO-A-01/21246
- US-A1- 2003 181 988
- US-A1- 2004 087 980

## Description

The invention relates to a surgical implant which is suitable in particular for the repair of inguinal hernias.

WO 92/19162 A2 discloses a hernia implant in which two areal parts are held at a distance by a cylindrical connection piece. The implant is applied with the aid of an insertion instrument with which the areal parts are compressed but which also makes control difficult during insertion of the implant. The connection piece has a predetermined length and for this reason does not adapt particularly well to irregular cavities of a body opening.

EP 0 815 794 A1 shows a stopper made of elastic material and of similar geometry which is used to close blood vessel walls and which is also applied with the aid of an insertion instrument.

EP 0 898 945 A1 describes a hernia implant with two conically shaped parts of areal material whose vertices coincide and which differ in terms of their aperture angle. This implant deforms when introduced into a hernial orifice, but it does not generally completely fill the hernial canal and requires additional fixing.

A areal hernia implant which can be deployed with the aid of a balloon in laparoscopic applications is disclosed in US 6 695 856 B2.

EP 1 145 693 A2 describes a hernia implant which is made of areal material and has the shape of a double cone with common base surface. A production method for such an implant is disclosed in US 6 712 859 B2. The implant is flexible and adapts to the local conditions when inserted into a hernial canal. Because of shape changes taking place under tensile loading, it is difficult to control the position of the deformed implant part during application, with the result that it is not always possible to reliably differentiate between a location in the hernial canal and in the preperitoneal space. Under compression loading, e.g. intra-abdominal pressure, as may occur, for example, when coughing, the implant may slip back out of the hernial orifice.

US 2004/087980 A1 describes an implantable prosthesis in which a tether is attached to a flat patch comprising a resilient annular support ring at its periphery. For repairing a hernia, an additional overlay patch can be used which comprises slots for guiding the tether.

US 2003/181988 A1 discloses a hernia repair device comprising two flexible mesh structures. Each of these mesh structures includes an essentially frustoconical, pleated first section and a second section inverted into the first section such that the second section is radially surrounded by the first section. Both meshes are connected to each other along the periphery of the base side of the frustoconical shapes such that a kind of double-frustoconical body is formed. By means of pulling threads, this body is expandable or collapsible in a radial direction so as to conform to the size and shape of a hernia defect. Moreover, the hernia repair device includes a generally flat overlay patch which is attached to the body by means of a fibre strand. When applied, the patch rests on one side of the bodily tissue containing the hernia defect, whereas the body fills the defect and, upon actuation of the pulling thread, broadens and flattens at the underside of the tissue, thus securing the hernia repair device. The presence of the overlay patch of the known hernia repair device sometimes results in a more complicated surgical procedure, with the adaption to the local anatomical conditions being troublesome sometimes.

The object of the invention is to make available a surgical implant which is suitable in particular for the repair of inguinal hernias, is easy to apply, adapts to the local anatomical conditions, and ensures a secure fit during the surgical intervention and also during the healing process.

This object is achieved by a surgical implant with the features of Claim 1. Advantageous embodiments of the invention are set out in the dependent claims.

The surgical implant according to the invention has an edge structure by means of which an inner area is defined. A flexible first mesh structure, which extends from the edge structure, is deformed three-dimensionally and spans the inner area of the edge structure. Moreover, a flexible second mesh structure extends from the edge structure, spans the inner area of the edge structure and is arranged opposite the first mesh structure. The second mesh structure is also deformed three-dimensionally. A pulling thread is also provided, which engages on the first mesh structure and is guided displaceably through the second mesh structure.

Moreover, the edge structure has a reinforcement ring. The reinforcement ring can be absorbable or non-absorbable, and is preferably absorbable. When, during application of the implant, the edge structure is guided through a hernial canal, the reinforcement ring should be flexible.

The edge structure is, for example, closed and shaped like a brim. In a preferred embodiment, the edge structure is formed by the edge areas of the first mesh structure and second mesh structure, which are connected to one another in their edge areas.

The first and second mesh structures can, for example, be conical, frustoconical, arched or pouch-like.

In a preferred embodiment of the invention, the two mesh structures are conical, and their common base defines the inner area of the edge structure. The edge structure extends radially outwards from said common base. Such an implant has similarities to the aforementioned implant according to EP 1 145 693 A2 and US 6 712 859 B2, but it additionally has the edge structure, which permits positional control of the implant during application and ensures a secure fit in the body tissue.

Generally speaking, no complicated instrumentation is needed for applying the surgical implant according to the invention. The implant can be fitted into a patient's hernial canal in open surgery, either by hand or with simple instruments, for example forceps or a needle holder. In doing so, the hernial canal, even when it is not round but instead has an irregular shape, is lined by the flexible mesh structures. The edge structure permits secure positioning and control during the operation. Moreover, during the healing process, a reliable fit of the implant is ensured even in cases of quite high intra-abdominal pressures. Fixation of the implant is not absolutely essential, because the implant is stable in position, by virtue of its shape, in particular because of the edge structure. As has been mentioned, the implant according to the invention is suitable for use in open surgery, but it can also be used, for example, in minimally invasive surgery with the aid of trocar sleeves.

In repair of an inguinal hernia, the edge structure, which is flexible, is guided through the hernial orifice so that it comes to lie in the preperitoneal space. The first mesh structure is also initially located there and, by means of a pull exerted on the pulling thread, it is then pulled out through the hernial orifice so that, together with the second mesh structure, it fills the hernial orifice and forms a kind of patchwork above the hernial orifice (under the skin). If the pulling thread is provided with a slip knot, the implant can be fixed by moving the slip knot in the direction towards the edge structure.

In principle, however, the implant according to the invention can be used not just in the preperitoneal space, as explained in connection with the above example, but also in intraperitoneal applications. In the latter case, adhesions with internal organs must be avoided (see below).

Depending on the configuration of the first mesh structure and second mesh structure, it may be advantageous if the implant has more than one pulling thread.

Moreover, the edge structure can be provided with means for anchoring in tissue, e.g. with barbs, in order to make it easier to fix the implant on the body tissue.

The first mesh structure and the second mesh structure can, in principle, be designed and produced by all manners known for implant meshes, e.g. as drawn-loop knits, formed-loop knits and/or crochet galloons. Designs in which the first mesh structure and/or the second mesh structure has a spacer knit are also advantageous. Three-dimensional deformation can take place, for example, by means of previously known heating methods.

The surgical implant according to the invention can contain absorbable material, non-absorbable material or a mixture of absorbable and non-absorbable materials.

In the latter case, for example when used as a hernia implant, the implant part to be introduced into the preperitoneal space can be made of non-absorbable material, while the implant part located above this is absorbable. All materials which can be employed for surgical implants (i.e. all biocompatible materials) can be used in principle. Particularly suitable non-absorbable materials are polypropylene (sold by Ethicon under the trade name "Prolene") and a mixture of polyvinylidene fluoride and a copolymer of vinylidene fluoride and hexafluoropropylene (sold by Ethicon under the trade name "Pronova"), and particularly suitable absorbable materials are a copolymer of glycolide and ε-caprolactone (Polyglecaprone 25, sold by Ethicon under the trade name "Monocryl"), a copolymer of glycolide and lactide in the ratio 90:10 (Polyglactin 910, sold by Ethicon under the trade name "Vicryl") and poly-p-dioxanone (PDS).

The invention is explained in more detail below on the basis of illustrative embodiments and with reference to the drawings, in which:
- Figure 1: shows, in parts (a) to (e), a schematic representation of an embodiment of the surgical implant according to the invention, and a schematic view of several steps in the application of the implant for repair of an inguinal hernia,
- Figure 2: shows, in parts (a) to (d), schematic representations of further embodiments of the implant according to the invention, the left-hand side in each case showing the implant before insertion into a hernial canal and the right-hand side in each case showing the implant after insertion into the hernial canal,
- Figure 3: shows a schematic representation of another embodiment of the implant according to the invention, the left-hand side showing the implant before insertion into a hernial canal and the right-hand side showing the implant after insertion into the hernial canal,
- Figure 4: shows, in parts (a) to (e), schematic side views of further embodiments of the implant according to the invention, and
- Figure 5: shows a schematic view of a formed-loop knit (according to Example 4) used in embodiments of the implant according to the invention.

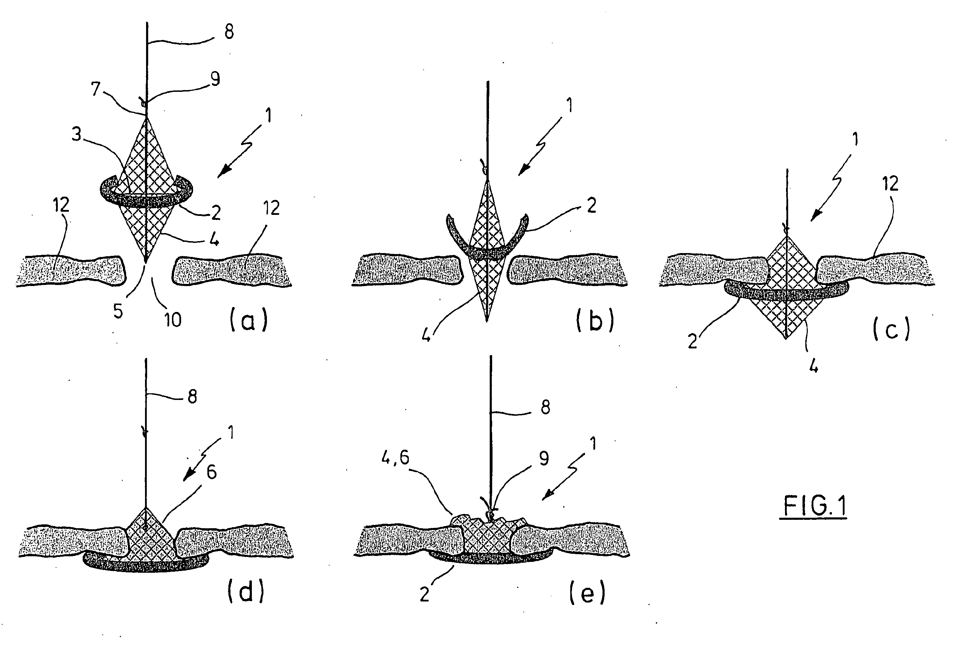

Figure 1, parts (a) to (e), shows several steps involved in the insertion of a surgical implant 1 into the orifice of an inguinal hernia.

The implant 1 to be applied is explained in more detail below with reference to Figure 1(a).

The implant 1 has an edge structure 2 which, in the illustrative embodiment, is designed as a flat circular ring, thus having an appearance similar to that of a brim. The edge structure 2 defines an inner area, namely the surface area located to the inside of the edge structure 2. This inner area 3 is spanned by a mesh structure 4 which is conically deformed three-dimensionally and extends downwards in the view in Figure 1(a) and ends with a tip 5. A second mesh structure 6 also extends from the edge structure 2 and likewise spans the inner area 3 and is designed as a cone with a tip 7. The second mesh structure 6 extends upwards, so that the implant 1 as a whole has the shape of a double cone, the parts of which have a common base from which the edge structure 2 extends outwards in the manner of a brim.

The two mesh structures 4 and 6 are flexible and can, for example, have a formed-loop knit or a spacer knit, as has already been mentioned and as will be explained in more detail below. The edge structure 2 can in this case be formed by the edge areas of the first mesh structure 4 and of the second mesh structure 6, which are connected to one another in their edge areas. Additionally, or alternatively, the edge structure 2 can have other material or can be formed in another way. Generally, in all embodiments, the edge structure has a reinforcement ring. In the illustrative embodiment, the edge structure 2 is flexible.

The implant 1 includes a pulling thread 8 which, in the illustrative embodiment, is guided through the second mesh structure 6 near the tip 7 and engages on the first mesh structure 4 near the tip 5. In the illustrative embodiment, however, the pulling thread 8 is not secured on the first mesh structure 4, but is instead guided displaceably, e.g. through openings on the first mesh structure 4, is turned back near the tip 5 and emerges likewise displaceably through the second mesh structure 6 near the tip 7, before ending with a slip knot 9.

In Figure 1(a), the implant 1 is shown before insertion into a hernial orifice (hernial canal) 10 which extends through body tissue 12 and opens into the preperitoneal area (in each case towards the bottom in Figure 1).

During an operation to close the hernial orifice 10, which is preferably performed by open surgery, the implant 1 is guided with the first mesh structure 4 through the hernial orifice 10, as is shown in Figure 1(b). In the process of doing this, the edge structure 2 bends so that the lower area of the implant 1 can be guided through the hernial orifice 10 and into the preperitoneal space. Figure 1(c) shows the state after the edge structure 2 has resumed its original shape and bears on the underside of the body tissue 12.

The operating surgeon then pulls on the pulling thread 8, as a result of which the first mesh structure 4 folds upwards and is drawn into the second mesh structure 6, see Figure 1(d). Thereafter, the two flexible mesh structures 4 and 6 can be pressed together, see Figure 1(e), by which means the hernial orifice 10 is filled. In this way, a kind of flange forms on the upper face of the body tissue 12 and, in cooperation with the edge structure 1, anchors the implant 1 securely in the hernial orifice 10. To fix it, use is made of the slip knot 9, which is moved downward with the aid of forceps for example, while the pulling thread 8 is drawn farther upwards. Finally, the pulling thread 8 is cut off above the slip knot 9.

The implant 1 is flexible, adapts well to the local conditions and reliably closes the hernial orifice 10. The preferably open-pore mesh structures 4 and 6 permit good incorporation of body tissue during the healing process.

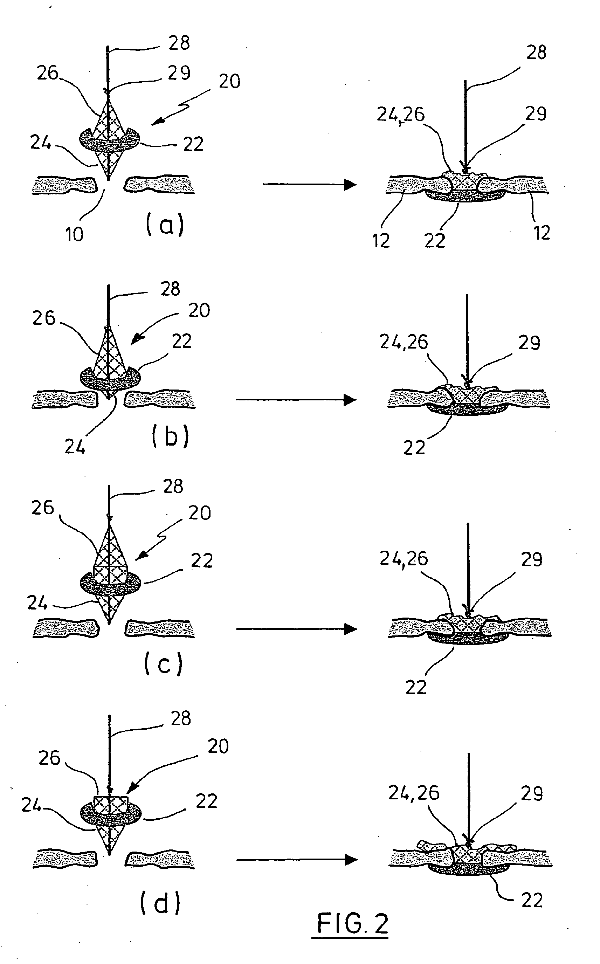

Figure 2, parts (a) to (d), shows further embodiments of the surgical implant, here designated by 20, the left-hand side in each case showing the implant before insertion into the hernial orifice 10 and the right-hand side in each case showing the implant after application. Since these embodiments differ from one another only slightly, the same reference numbers are used for the sake of simplicity.

The implants 20 have in each case an edge structure 22, a first mesh structure 24, a second mesh structure 26, and a pulling thread 28 whose end is secured with a slip knot 29. The embodiment according to Figure 2(a) is largely similar to the embodiment according to Figure 1. In the embodiments according to Figure 2(b) and Figure 2(c), the second mesh structure 26 is larger than the first mesh structure 24, while in the embodiment according to Figure 2(d) the second mesh structure 26 does not have a conical shape. The implant is applied in the manner described with reference to Figure 1.

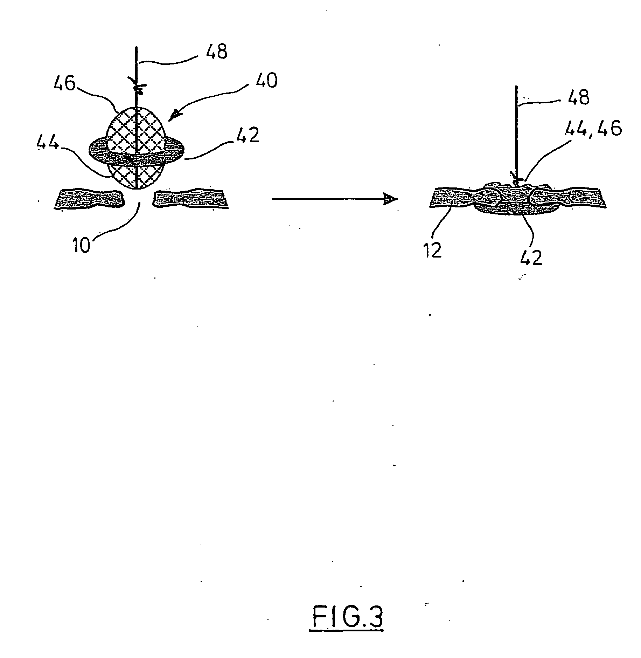

Figure 3 shows a schematic representation of another embodiment of the surgical implant, the left-hand side again showing the implant before insertion into a hernial orifice 10 and the right-hand side showing the implant after insertion into the hernial orifice 10, which is delimited by body tissue 12. However, this embodiment differs more from the ones previously described.

The implant 40 according to Figure 3 includes an edge structure 42 from which there extend a first mesh structure 44 in the shape of a hemisphere and a second mesh structure 46 in the shape of a hemisphere. Again, a pulling thread 48 with a slip knot is provided.

Here, as in the other embodiments too, the pulling thread can alternatively be fixed on the first mesh structure.

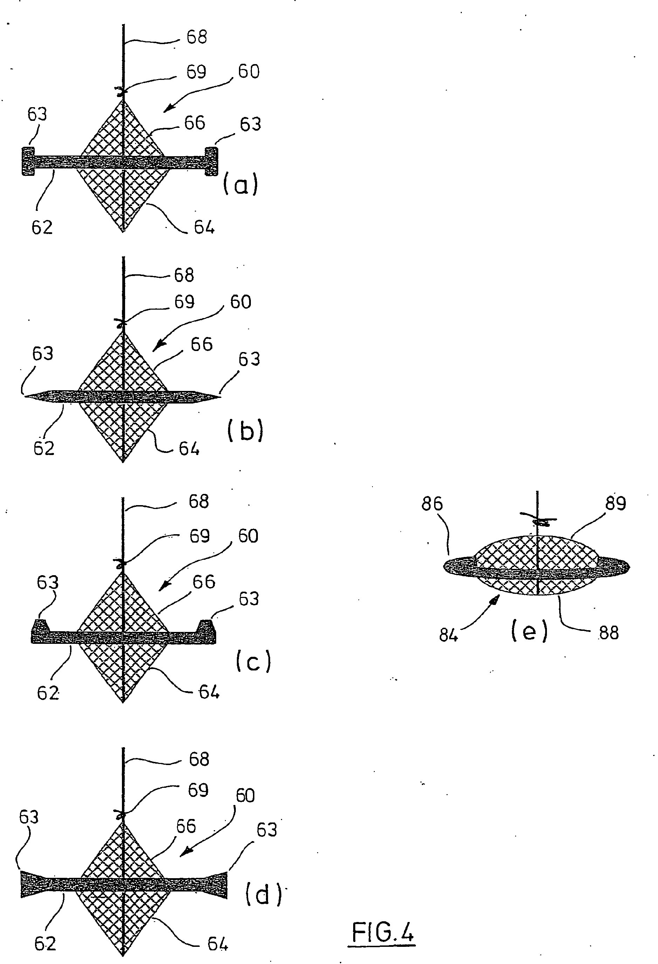

Figure 4, parts (a) to (e), shows further embodiments of the surgical implant in schematic representations.

The embodiments according to Figures 4(a) to 4(d) are once again fairly similar to one another, for which reason the same reference numbers are used for them. The implants 60 each have an edge structure 62 with, in the illustrative embodiment, a circular periphery 63, a first mesh structure 64, a second mesh structure 66, and a pulling thread 68 with a slip knot 69, similarly as described above. However, the periphery 63 in these cases has a particular configuration, specifically, according to Figures 4(a), 4(c) and 4(d), as a reinforcement ring which, in the embodiment according to Figure 4(d), can additionally fulfil a kind of barb function, and, according to Figure 4(b), as a cutting edge which permits better fixing of the edge structure 62 in the body tissue. In all cases, the edge structure 62 can be produced as a suitably shaped separate part which is connected to the first mesh structure 64 and to the second mesh structure 66. The same also applies analogously to the embodiment according to Figure 4(e).

The implant 84 according to Figure 4(e) has a design similar to the implant 40 according to Figure 3, but, in contrast to the latter, it has an elliptic edge structure 86 and correspondingly shaped mesh structures 88 and 89.

The embodiments described with reference to Figure 4 are applied in a similar manner as the embodiments discussed above, with additional fixing being achieved in some cases through the particular shape of the edge structure.

There follow a number of examples of how the first and second mesh structures are produced and of how a three-dimensional implant can be formed from these.

### Example 1

To produce a mesh structure configured as a partially absorbable spacer knit, non-absorbable threads ("Pronova", see above, 110 dtex) and absorbable threads ("Monocryl", see above, 200 dtex) were worked together on a double-bar crochet galloon machine from the company called Comez (4-feed). The thread sheet comprised untwisted warps of non-absorbable material ("Pronova") and absorbable material ("Monocryl").

To obtain a spacer knit with an open-pore front in filet structure and a closed reverse, the material was worked with several thread sheets according to the following pattern:
Bar 1: 1-0/0-0/1-2/2-2/1-0/0-0/1-2/2-2/2-3/3-3/2-1/1-1/2-3/3-3/2-1/1-1//
Bar 2 : 2-3/3-3/2-1/1-1/2-3/3-3/2-1/1-1/1-0/0-0/1-2/2-2/1-0/0-0/1-2/2-2//
Bar 3 : 0-1/1-0/1-2/1-2/1-0/1-0/1-2/1-2/3-2/3-2/1-2/1-2/3-2/3-2/1-2/2-1//
Bar 4 : 2-3/2-3/2-1/2-1/2-3/2-3/2-1/2-1/0-1/0-1/2-1/2-1/0-1/0-1/2-1/2-1//
Bar 5 : 4-4/4-0/0-0/0-4//
Bar 6 : 0-1/1-1/1-0/0-0//

In this pattern, after absorption, the remaining non-absorbable portion amounts to ca. 20% of the original weight. To reduce the absorbable portion by ca. 50%, production of a spacer knit with bars 1, 2, 4 and 6 is possible.

### Example 2

With polypropylene monofilaments ("Prolene", see above, 3.5 mils = 0.089 mm diameter) and absorbable "Monocryl" (see above, 200 dtex) as starting materials, a spacer knit with a structure closed on both sides was worked according to the following pattern:
Bar 1: 4-4/4-0/0-0/0-4//
Bar 2: 0-1/1-1/1-0/0-0//
Bar 3: 2-3/1-2/1-0/2-1//
Bar 4: 0-1/1-2/3-2/2-1//
Bar 5: 0-0/0-1/1-1/1-0//
Bar 6: 4-0/0-0/0-4/4-4//

After absorption, the remaining non-absorbable portion amounts to ca. 20% of the original weight. To reduce the absorbable portion by ca. 50%, production of a spacer knit with bars 1, 2, 4 and 6 is also possible here.

### Example 3

With the same starting materials as in Example 1, a spacer knit with an open filet structure on both sides was worked according to the following pattern:
Bar 1 : 1-0/0-0/1-2/2-2/1-0/0-0/1-2/2-2/2-3/3-3/2-1/1-1/2-3/3-3/2-1/1-1//
Bar 2 : 2-3/3-3/2-1/1-1/2-3/3-3/2-1/1-1/1-0/0-0/1-2/2-2/1-0/0-0/1-2/2-2//
Bar 3 : 0-1/1-0/1-2/1-2/1-0/1-0/1-2/1-2/3-2/3-2/1-2/1-2/3-2/3-2/1-2/2-1//
Bar 4 : 2-3/2-3/2-1/2-1/2-3/2-3/2-1/2-1/0-1/0-1/2-1/2-1/0-1/0-1/2-1/2-1//
Bar 5: 2-2/1-0/0-0/1-2//
Bar 6: 0-0/1-2/2-2/1-0//

Once again, after absorption of the "Monocryl" portion, a remaining "Pronova" portion of ca. 20% is obtained. To reduce the absorbable portion by ca. 50%, production of a spacer knit with bars 1, 4 and 6 is possible here.

### Example 4

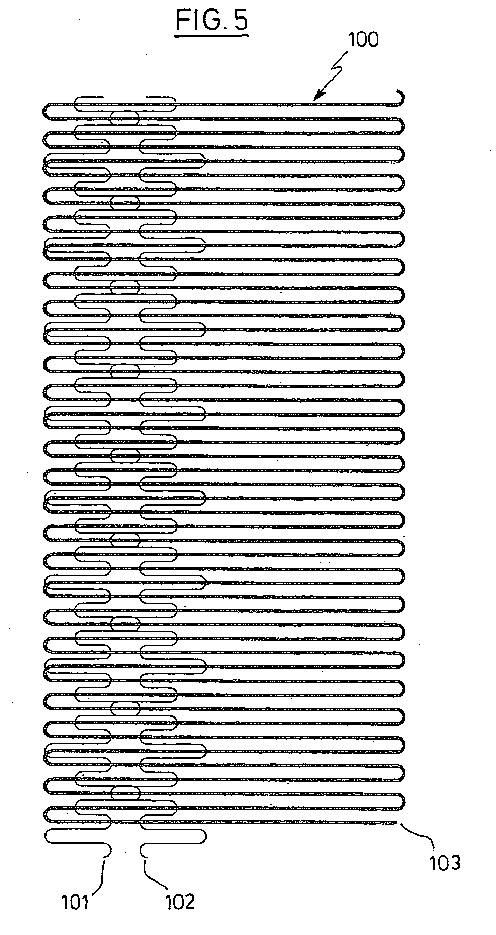

To produce the partially absorbable knit 100 shown schematically in Figure 5 with reinforcement threads, non-absorbable polypropylene threads ("Prolene" see above, 65 dtex) and absorbable threads ("Monocryl", see above, 150 dtex) were worked on a crochet galloon machine (6-feed) from the company called Comez. The thread sheet comprised a non-absorbable warp and partially absorbable, or absorbable, wefts. The warp gave a closed fringe.

The other threads were divided into three partial wefts:

| | | |
|---|---|---|
| Partial weft I: | 2-4/2-3/1-3// | (reference 101) |
| Partial weft II: | 3-1/3-2/4-2// | (reference 102) |
| Partial weft III: | 1-11// | (reference 103) |

The threads of partial wefts I and II comprised twists with in each case one "Prolene" thread and one "Monocryl" thread. These were produced on a Lezzini two-stage twister machine with 174S/155Z turns. The partial weft III comprised an absorbable twist. For this purpose, two "Monocryl" threads were twisted on a Lezzini two-stage twister machine. Further twist variants for partial weft III are:
- 3x "Monocryl"
- 4x "Monocryl"

To obtain a permanent reinforcement of the mesh structures of the implant, twists can be produced with the following materials:
- 1x "Prolene" + 3x "Monocryl"
- 1x "Prolene" + 4x "Monocryl"

### Example 5

A formed-loop knit produced as in Example 4 was additionally linked (e.g. sewn) to a commercially available knit made of ORC (oxidized regenerated cellulose, trade name "Interceed").

A flexible mesh structure formed in this way can be used as part of an implant also in the intraperitoneal space, because adhesions of the intestine or of the internal organs on the implant can be avoided by means of the ORC component.

### Example 6

To produce an implant according to Figure 1 or Figure 2, the mesh structure variants produced according to Examples 1 to 5 were cleaned in an ethyl acetate bath and then stretched on a frame and thermally fixed for 10 hours under dry inert gas at ca. 110°C.

For further processing, the respective mesh structure was thermally treated a second time. For this purpose, the mesh structure was fixed in the desired three-dimensional shape for a period of 10 minutes at ca. 140°C.

The material was then cut with punching dies to give a first mesh structure 4 or 24 and a second mesh structure 6 or 26 of the desired size. A first mesh structure 4 or 24 and a second mesh structure 6 or 26 were then sewn together in their edge zones with non-absorbable "Pronova" sewing material, so that the brim-like edge structure 2 or 22 was formed.

A biocompatible suture thread (pulling thread 8 or 28) was applied to the structure so that a surgeon, using slip knot 9 or 29, is able to compress the two three-dimensional mesh structures 4 or 24 and 6 or 26 and shape them according to the anatomical conditions, as has been explained above. This biocompatible suture thread preferably comprises non-absorbable monofilaments and/or multifilaments.

## Claims

1. Surgical implant, comprising
- an edge structure (2; 22; 42; 62; 86) by means of which an inner area (3) is defined,
- a flexible first mesh structure (4; 24; 44; 64; 88) which extends from the edge structure, is deformed three-dimensionally and spans the inner area of the edge structure,
- a flexible second mesh structure (6; 26; 46; 66; 89) which extends from the edge structure, is deformed three-dimensionally, spans the inner area of the edge structure and is arranged opposite the first mesh structure, and
- a pulling thread (8; 28; 48; 68) which engages on the first mesh structure and is guided displaceably through the second mesh structure
**characterized in that** the edge structure has a reinforcement ring.

2. Surgical implant according to Claim 1, **characterized in that** the reinforcement ring is absorbable.

3. Surgical implant according to Claim 1 or 2, **characterized in that** the edge structure (2; 22) is formed by the edge areas of the first mesh structure (4; 24) and second mesh structure (6; 26), which are connected to one another in their edge areas.

4. Surgical implant according to one of Claims 1 to 3, **characterized in that** the second mesh structure (6; 26; 46; 66; 89) has one of the shapes selected from the following list: conical, frustoconical, arched, pouch-like.

5. Surgical implant according to one of Claims 1 to 4, **characterized in that** the first mesh structure (4; 24; 44; 64; 88) has one of the shapes selected from the following list: conical, frustoconical, arched, pouch-like.

6. Surgical implant according to one of Claims 1 to 5, **characterized in that** the edge structure (2; 22; 42; 62; 86) is closed and shaped like a brim.

7. Surgical implant according to one of Claims 1 to 6, **characterized in that** the edge structure (62) has means for anchoring in tissue, preferably barbs.

8. Surgical implant according to one of Claims 1 to 7, **characterized in that** more than one pulling thread is provided.

9. Surgical implant according to one of Claims 1 to 8, **characterized by** a slip knot (9; 29; 69) which is displaceable on a pulling thread (8; 28; 48; 68).

10. Surgical implant according to one of Claims 1 to 9, **characterized in that** the first mesh structure (4; 24; 44; 64; 88) and/or the second mesh structure (6; 26; 46; 66; 89) has a knit and/or a spacer knit.

11. Surgical implant according to one of Claims 1 to 10, **characterized by** absorbable material, preferably at least one of the materials selected from the following list: absorbable natural polymers, collagens, absorbable synthetic polymers, polyhydroxy acids, polylactides, polyglycolides, polycaprolactones, polydioxanones, polyether esters, copolymers and mixtures of such substances.

12. Surgical implant according to one of Claims 1 to 11, **characterized by** non-absorbable material, preferably at least one of the materials selected from the following list: non-absorbable polymers, polyolefins, polypropylene, fluorine-containing polyolefins, mixtures of polyvinylidene fluoride and copolymers of vinylidene fluoride and hexafluoropropylene, polyesters, polyamides.

## Patentansprüche

1. Chirurgisches Implantat, mit
- einer Randstruktur (2; 22; 42; 62; 86), durch die ein Innenbereich (3) definiert ist,
- einer flexiblen ersten Netzstruktur (4; 24; 44; 64; 88), die von der Randstruktur ausgeht, ins Dreidimensionale verformt ist und den Innenbereich der Randstruktur überspannt,
- einer flexiblen zweiten Netzstruktur (6; 26; 46; 66; 89), die von der Randstruktur ausgeht, ins Dreidimensionale verformt ist, den Innenbereich der Randstruktur überspannt und gegenüber der ersten Netzstruktur angeordnet ist, und
- einem Zugfaden (8; 28; 48; 68), der an der ersten Netzstruktur angreift und verschiebbar durch die zweite Netzstruktur hindurch geführt ist,
**dadurch gekennzeichnet, dass** die Randstruktur einen Verstärkungsring aufweist.

2. Chirurgisches Implantat nach Anspruch 1, **dadurch gekennzeichnet, dass** der Verstärkungsring resorbierbar ist.

3. Chirurgisches Implantat nach Anspruch 1 oder 2, **dadurch gekennzeichnet, dass** die Randstruktur (2; 22) durch die Randbereiche der ersten Netzstruktur (4; 24) und der zweiten Netzstruktur (6; 26) ausgebildet ist, die in ihren Randbereichen miteinander verbunden sind.

4. Chirurgisches Implantat nach einem der Ansprüche 1 bis 3, **dadurch gekennzeichnet, dass** die zweite Netzstruktur (6; 26; 46; 66; 89) eine der aus der folgenden Liste ausgewählten Formen hat: kegelartig, kegelstumpfartig, gewölbt, beutelartig.

5. Chirurgisches Implantat nach einem der Ansprüche 1 bis 4, **dadurch gekennzeichnet, dass** die erste Netzstruktur (4; 24; 44; 64; 88) eine der aus der folgenden Liste ausgewählten Formen hat: kegelartig, kegelstumpfartig, gewölbt, beutelartig.

6. Chirurgisches Implantat nach einem der Ansprüche 1 bis 5, **dadurch gekennzeichnet, dass** die Randstruktur (2; 22; 42; 62; 86) in sich geschlossen und krempenartig ausgestaltet ist.

7. Chirurgisches Implantat nach einem der Ansprüche 1 bis 6, **dadurch gekennzeichnet, dass** die Randstruktur (62) Gewebeverankerungsmittel aufweist, vorzugsweise Widerhaken.

8. Chirurgisches Implantat nach einem der Ansprüche 1 bis 7, **dadurch gekennzeichnet, dass** mehr als ein Zugfaden vorgesehen ist.

9. Chirurgisches Implantat nach einem der Ansprüche 1 bis 8, **gekennzeichnet durch** einen Schiebeknoten (9; 29; 69), der auf einem Zugfaden (8; 28; 48; 68) verschiebbar ist.

10. Chirurgisches Implantat nach einem der Ansprüche 1 bis 9, **dadurch gekennzeichnet, dass** die erste Netzstruktur (4; 24; 44; 64; 88) und/oder die zweite Netzstruktur (6; 26; 46; 66; 89) ein Gewirke und/oder ein Abstandsgewirke aufweist.

11. Chirurgisches Implantat nach einem der Ansprüche 1 bis 10, **gekennzeichnet durch** resorbierbares Material, vorzugsweise mindestens eines der aus der folgenden Liste ausgewählten Materialien: resorbierbare natürliche Polymere, Kollagene, resorbierbare synthetische Polymere, Polyhydroxysäuren, Polylactide, Polyglykolide, Polycaprolactone, Polydioxanone, Polyetherester, Copolymere und Mischungen derartiger Substanzen.

12. Chirurgisches Implantat nach einem der Ansprüche 1 bis 11, **gekennzeichnet durch** nichtresorbierbares Material, vorzugsweise mindestens eines der aus der folgenden Liste ausgewählten Materialien: nichtresorbierbare Polymere, Polyolefine, Polypropylen, fluorhaltige Polyolefine, Mischungen aus Polyvinylidenfluorid und Copolymeren aus Vinylidenfluorid und Hexafluorpropen, Polyester, Polyamide.

## Revendications

1. Implant chirurgical, comprenant :
◆ une structure d'arête (2 ; 22 ; 42 ; 62 ; 86) au moyen de laquelle une surface interne (3) est définie,
◆ une première structure de maille flexible (4 ; 24 ; 44 ; 64 ; 88) qui s'étend à partir de la structure d'arête, est déformée au plan tridimensionnel et couvre la surface interne de la structure d'arête,
◆ une seconde structure de maille flexible (6 ; 26 ; 46 ; 66 ; 89) qui s'étend à partir de la structure d'arête, est déformée au plan tridimensionnel, couvre la surface interne de la structure d'arête et est disposée à l'opposé de la première structure de maille, et
◆ un fil de traction (8 ; 28 ; 48 ; 68) qui s'engage sur la première structure de maille et est guidé de façon déplaçable à travers la seconde structure de maille,
**caractérisé en ce que** la structure d'arête a une bague de renforcement.

2. Implant chirurgical selon la revendication 1, **caractérisé en ce que** la bague de renforcement est absorbable.

3. Implant chirurgical selon la revendication 1 ou 2, **caractérisé en ce que** la structure d'arête (2 ; 22) est formée par les surfaces d'arête de la première structure de maille (4 ; 24) et de la seconde structure de maille (6 ; 26), qui sont reliées l'une à l'autre dans leurs surfaces d'arête.

4. Implant chirurgical selon l'une des revendications 1 à 3, **caractérisé en ce que** la seconde structure de maille (6 ; 26 ; 46 ; 66 ; 89) a l'une des formes choisies parmi la liste suivante : conique, frustoconique, arquée, en forme de bourse.

5. Implant chirurgical selon l'une des revendications 1 à 4, **caractérisé en ce que** la première structure de maille (4 ; 24 ; 44 ; 64 ; 88) a l'une des formes choisies parmi la liste suivante : conique, frustoconique, arquée, en forme de bourse.

6. Implant chirurgical selon l'une des revendications 1 à 5, **caractérisé en ce que** la structure d'arête (2 ; 22 ; 42 ; 62 ; 86) est fermée et en forme de bord.

7. Implant chirurgical selon l'une des revendications 1 à 6, **caractérisé en ce que** la structure d'arête (62) a des moyens d'ancrage dans les tissus, de préférence des barbelés.

8. Implant chirurgical selon l'une quelconque des revendications 1 à 7, **caractérisé en ce que** plus d'un fil de traction est fourni.

9. Implant chirurgical selon l'une des revendications 1 à 8, **caractérisé par** un noeud en bec d'oiseau (9 ; 29 ; 69) qui est déplaçable sur un fil de traction (8 ; 28 ; 48 ; 68).

10. Implant chirurgical selon l'une des revendications 1 à 9, **caractérisé en ce que** la première structure de maille (4 ; 24 ; 44 ; 64 ; 88) et/ ou la seconde structure de maille (6 ; 26 ; 46 ; 66 ; 89) a un tricotage et/ou un tricotage d'espacement.

11. Implant chirurgical selon l'une des revendications 1 à 10, **caractérisé par** un matériau absorbable, de préférence au moins un des matériaux choisis parmi la liste suivante : polymères naturels absorbables, collagènes, polymères de synthèse absorbables, acides polyhydroxy, polylactides, polyglycolides, polycaprolactones, polydioxanones, esters de polyéther, copolymères et mélanges de ces substances.

12. Implant chirurgical selon l'une des revendications 1 à 11, **caractérisé par** un matériau non-absorbable, de préférence au moins un des matériaux choisis parmi la liste suivants : polymères non-absorbables, polyoléfines, polypropylène, polyoléfines contenant du fluor, mélanges de polyfluorure de vinylidène et copolymères de fluorure de vinylidène et d'hexafluoropropylène, polyesters, polyamides.
